Europäisches Patentamt

European Patent Office

Office européen des brevets

(11) Publication number: **0 463 222 A2**

## EUROPEAN PATENT APPLICATION

(21) Application number: **90118478.8**

(22) Date of filing: **26.09.90**

(51) Int. Cl.5: **G01N 33/569**, C12Q 1/04, G01N 33/68, G01N 33/53, G01N 33/66

(30) Priority: **28.06.90 US 544979**

(43) Date of publication of application:
**02.01.92 Bulletin 92/01**

(84) Designated Contracting States:
**AT BE CH DE DK ES FR GB GR IT LI LU NL SE**

(71) Applicant: **VICAM, L.P.**
**29 Mystic Avenue**
**Sommerville, Massachusetts 02145(US)**

(72) Inventor: **Green, Calvert L.**
**16 Brookside Lane**
**Norfolk, Massachusetts 02056(US)**
Inventor: **Hansen, Thomsen J.**
**197 Fuller Street**
**Brookline, Massachusetts 02146(US)**
Inventor: **Tannenbaum, Steven R.**
**14 Hickey Drive**
**Framingham, Massachusetts 01701(US)**
Inventor: **Livingston, David M.**
**11 Powell Street**
**Brookline, Massachusetts 02146(US)**
Inventor: **Portney, Daniel A.**
**4512 Regent Street**
**Philadelphia, Pennsylvania 19143(US)**
Inventor: **Wogan, Gerald N.**
**125 Claflin Street**
**Belmont, Massachusetts 02178(US)**
Inventor: **Benjamin, Thomas L.**
**595 Putnam Avenue**
**Cambridge, Massachusetts 02139(US)**

(74) Representative: **Vossius & Partner**
**Siebertstrasse 4 P.O. Box 86 07 67**
**W-8000 München 86(DE)**

(54) **Assay method for detecting viable listeria.**

(57) An assay method is provided to quickly detect the presence of Listeria strains in samples, characterized by the selective growth of Listeria cells within macrophage-like cells and subsequent detection of the amplified Listeria cells or components of the Listeria bacterial cell wall.

This invention relates to an assay method for quickly and easily detecting the presence of bacteria of the genus Listeria. In particular, an immunoassay method is utilized to detect the presence of viable Listeria strains in foods and other potentially contaminated samples using an assay characterized by 1) selective growth of Listeria cells within macrophage-like cells, and 2) subsequent detection of the amplified Listeria cells or cellular products.

The presence of bacterial pathogens is a well recognized cause of severe illness, so that there is an ever present need for the detection of such pathogens in both clinical specimens (i.e. blood, tissue, urine and other body extracts and fluids) and agricultural specimens (such as food products).

However, current tests for the detection of bacterial pathogens, such as in food, typically require a number of days to complete. During this period of time, between sampling and assay determination, fresh food and dairy products will enter the food chain and therefore be consumed by the public. If a test indicates the presence of pathogens, expensive product recalls may result, or, worse, before the test results are discovered an outbreak of sickness may occur.

This was exactly the case in a number of recent outbreaks of Listeria in fresh dairy and vegetable products. In the case of Listeria, an outbreak generally produces a death rate greater than 40%. The death rate is much higher for newborns, pregnant women, the elderly and immunocompromised individuals. Spontaneous abortions result even in otherwise asymptomatic patients. In fact, it is estimated that as many as 2% of all spontaneous abortions may be due to Listeria infection.

As stated above, traditional methods to detect the presence of bacterial food pathogens require an extended period of time, basically due to the need for an incubation period. This incubation period is intended to allow for recovery of injured bacteria, growth of these bacteria from a background of competing microorganisms and an increase in bacterial cell numbers to more readily aid in identification. In many cases a series of two or three separate incubations is needed to isolate the target bacteria.

In the standard FDA procedure for detection of Listeria in food products (Bacteriological Analytical Manual, 6th ed., 1984; Supplement, September 1987, Chapter 29) 25 g or 25 ml of a food sample is mixed with 225 ml of enrichment broth. This sample in broth mixture is incubated for 7 days. At the end of days 1 and 7 a sample of the broth culture is streaked onto petri plates containing selective growth agar and these plates are incubated for an additional 2 days. Identification of Listeria colonies confirms the presence of Listeria in the original food sample. This identification, however, is subjective, and therefore prone to misinterpretation, and this procedure requires a minimum of 7 days to confirm Listeria negative samples.

More recent methods of bacterial detection in food products have utilized immunoassays. Antibodies to an antigen present in the bacteria of interest are generally used in these methods in some form of a two site assay. That is, one antibody is immobilized and acts to capture the target bacterial antigens. This allows for separation of the target antigen from the food sample. A second antibody to this antigen (having the same or a different epitope) is labeled in some fashion such as radioactively with $^{125}$I or enzymatically with horse radish peroxidase, and when added to the immobilized antibody antigen complex also becomes immobilized. Subsequent steps remove unbound labeled antibody. The label left attached is measured and usually compared against standards (positive and negative controls) to determine the presence of the target bacteria. At least one of the two antibodies used in the two site assay must be specific to the target bacteria. This type of immunoassay is known as a direct assay. Other forms, such as the competition assay are also used but tend to be less sensitive, and technically more difficult to perform.

Because of the actual sensitivity limit of these assays it remains necessary to culture the target bacteria from the food sample. In some of the newer immunoassay tests incubation times have been reduced and the number of separate incubation steps have also been reduced. The resulting tests, however, still require about 48 hours to complete.

In a generalized "rapid" immunoassay for detection of Listeria, 25 g or 25 ml of food sample is mixed with 225 ml of enrichment broth, as in the FDA procedure. This culture mixture is incubated for 24 hours. From this 250 ml of culture, 1 ml is removed and added to 9 ml of selective growth medium. This selective culture mixture is incubated for an additional approximately 24 hours. At this point, depending upon the actual immunoassay format, some fraction of the total 10 ml subculture (usually 0.2 ml - 1.0 ml) is tested by immunoassay for the presence of Listeria.

In summary, these existing "rapid" immunoassay procedures for bacterial detection in food samples all require at least one (usually two or more) dilution of sample (into growth medium) followed by an assay procedure which only utilizes a fraction of this final culture. The actual assay sample thus only corresponds to a small fraction of the original food sample. The bacterial culture step, or steps, must therefore overcome this dilution factor, adding to the amount of needed culture time.

These and other disadvantages of the prior art methods are overcome by the present invention which provides a fast and accurate method for the detection of viable Listeria.

It is therefore one object of the present invention to provide a method for quickly detecting the presence of potentially pathogenic Listeria:

It is another object of the invention to provide a method for the detection of viable Listeria bacteria which requires a short and selective culture step from Listeria directly concentrated from the original sample.

These and other objects of the invention are accomplished by providing a method which selectively removes Listeria bacteria from a sample by the use of an antibody, selectively grows these Listeria bacteria within macrophage-like cells (or any other cell line capable of phagocytically ingesting Listeria) and, after releasing the Listeria from these cells, detects their presence with a labeled antibody or group of antibodies specific to Listeria bacteria.

Alternatively, detection of Listeria mediated polymerised cellular actin or Listeria hemolysin may be used.

As noted above, one object of the present invention is to provide an assay procedure for rapid identification of the presence of Listeria bacteria in food samples requiring only a short and selective culturing of the Listeria. This allows for interpretation of test results prior to release of fresh food products. The present invention overcomes the dilution factor effect by concentrating the bacteria onto the solid support directly from liquid, or liquified food samples. Once concentrated and separated from the food sample, further steps are used to detect the captured Listeria bacterial cells.

After the Listeria bacteria are immobilized and concentrated according to the invention they are added to an actively growing culture of macrophage-like cells attached to microscopic cover slips or wells of a microtiter plate. After a short incubation step (one hour or less) to allow the macrophage-like cells to phagocytize the Listeria bacteria, a cell growth media containing bacterial antibiotics is added to kill bacteria not internalized by the macrophage-like cells. Listeria bacterial cells, which secrete an enzyme, hemolysin, are then capable of dissolving the vacuolar membrane of the macrophage-like cells and entering the cells cytoplasm. Once in the cytoplasm the Listeria bacteria are capable of growing and reproducing at a rate equal to that seen in rich liquid bacterial growth media. Within 8 hours post ingestion the Listeria bacterial cell number can increase by 1000 times. In addition, an unknown Listeria bacterial cell product results in the polymerization of cellular actin within the macrophage-like cells. This polymerized actin is then organized into filaments and subsequently these filaments aggregate at one end of the Listeria cells thus pushing the Listeria cells forward. This process becomes the vehicle to transfer Listeria bacteria to adjacent macrophage-like cells (Tilney, L.G. and Portnoy, D.A. (1989), J. of Cell Biol., 109, 1597-1608).

The targets for detection of the Listeria cells can therefore be the amplified Listeria bacterial cells, the Listeria organized filaments of cellular actin or the hemolysin released by the Listeria bacteria to disrupt the vacuolar membranes of the cells.

Selectivity of the assay for Listeria bacteria results from the specificity of the antibodies used, the specificity of growth within the macrophage-like cells and transfer of Listeria between cells, and the specificity of the actin polymerization and organization reactions.

The method of the invention, therefore, generally comprises the following procedural steps:

1) The sample to be tested is, if necessary, liquified.

2) The liquid sample is combined with a solid matrix having immobilized thereon monoclonal or polyclonal antibodies to the target Listeria bacterial cells. If present, the target Listeria bacterial cells are thereby immobilized to the solid matrix. The solid matrix with immobilized bacteria attached is then washed to remove the remainder of the food sample.

3) The antibody immobilized Listeria bacterial cells are then released from the immobilizing matrix unless the solid support consists of microspheres.

4) The released Listeria bacterial cells or microsphere bound Listeria cells are then added to a microculture of macrophage-like cells attached to glass or plastic.

5) After a short incubation period to allow the macrophage-like cells to phagocytize the Listeria bacterial cells, the growth media is removed and replaced with media containing bacterial antibiotics, or antibiotics may be added directly to the growth medium. This step removes bacteria not internalized by the macrophage-like cells and also kills any external bacteria not removed with the original cell growth media.

If microspheres of antibody coated magnetic particles are the solid support, the Listeria bacteria cells bound to these particles can be more effectively brought into contact with the glass or plastic attached macrophage-like cells by placing the attached macrophage-like cells between the magnetic particle bound Listeria bacteria and a magnetic field during a short initial incubation period.

6) Once inside the macrophage-like cell vacuoles the Listeria bacteria secrete an enzyme (hemolysin) which digests the vacuolar membrane and releases the Listeria into the cellular cytoplasm. The Listeria bacteria now begin to grow and reproduce. Listeria cell growth within the macrophage-like cells is very efficient with a doubling time of approximately 50 minutes. Thus, in 8 hours after ingestion by the macrophage-like cells the Listeria bacteria have increased by a factor of 1000.

7) The culture media is now removed leaving the macrophage-like cells attached to the glass or plastic surface and the Listeria bacteria within the cellular cytoplasm.

8) The macrophage-like cells are now disrupted (by adding water or methanol) and the presence of the Listeria bacteria are detected by labeled antibodies to Listeria cells, by detection of organized filaments of polymerized actin, or by detection of Listeria secreted hemolysin.

As can be seen from the above method scheme, the method of the invention is particularly characterized by the use of an antibody/s to first select out Listeria bacteria from a sample. The antibody/s used for this step need not be totally specific to Listeria as subsequent selective and specific steps will result in the ultimate specificity of the assay for only potentially pathogenic Listeria bacteria. Further uniquely characterizing the method of the invention is the use of macrophage-like cells which represent an intracellular growth medium selective for potentially pathogenic Listeria. Using these characteristic features, the method of the invention can be importantly applied to the rapid and specific detection of viable and potentially pathogenic strains of Listeria bacteria.

Example for Detection of Potentially Pathogenic Strains of Listeria Using Macrophage-Like Cell Microculture

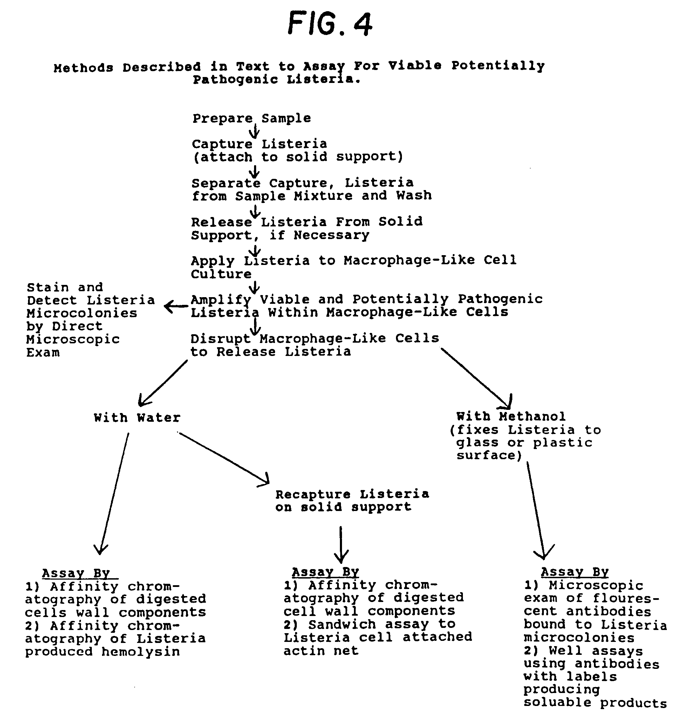

This general description of the method is summarized in outline form in Figure 4.

Step 1: Liquefaction

The assay of the invention can be utilized to detect the presence of Listeria in a wide variety of samples, both solid and liquid, including food, agricultural products, and various clinical specimens. If the sample is liquid, it can be per se subjected to the procedure of the invention, or first diluted. On the other hand, if the sample is solid, it should be first liquified (for example in water) using standard known techniques, such as by use of a blender. The liquid or liquified sample is filtered through a course paper, glass or other matrix filter to remove particulates. If the preferred method of the invention is used (capture of Listeria bacterial cells with antibody bound magnetic microspheres) this filtration step to remove particulates is not always required. If the sample to be tested is an environmental sample, then swabs or scrapings of the tested surface are mixed in a collection buffer and then treated as a liquified food sample.

Step 2: Immobilization With Antibodies

Immobilized antibodies (polyclonal or monoclonal) to target bacterial cells are used to separate Listeria cells from the sample. In this step, one or more antibodies must be used to recognize all target strains of the genus Listeria. Recognition of other bacteria (i.e. cross-reactivity) can be accepted in this step, as complete specificity is not necessary at this step of the assay.

The antibodies used in Step (1) recognize Listeria cell surface antigens, and these antibodies are immobilized on the preferred method microspheres which can be mixed with the sample and separated from the sample mixture in some manner subsequent to capture of the target Listeria bacterial cells. In particular, microspheres of magnetic particles may be used as the immobilizing matrix. After an incubation period in which the liquid sample or liquified sample is mixed with the magnetic particles having antibodies attached, the antibody bound particles and attached Listeria bacteria are separated from the sample by means of a magnetic field. Other beads, with antibodies attached, such as latex, glass, cross-linked dextrans, agarose and polysaccharides may be used to capture the Listeria bacteria cells followed by separation from the sample mixture by centrifugation or filtration. Also filtration of the sample after Listeria bacterial cell capture over columns packed with these beads may be used to separate and concentrate the Listeria from the sample mixture. In addition, filtration of the sample over cellulose nitrate or acetate, glass fiber or nylon membranes having anti-Listeria antibodies attached may be used to separate the Listeria cells from the sample mixture.

As the antibodies for capture, and potentially detection, of the Listeria bacteria, either polyclonal antibodies or monoclonal antibodies can be used depending upon various factors, including the degree of sensitivity desired. If polyclonal antibodies are to be used, then such antibodies can be prepared according to per se known procedures. For example, procedures such as those described by Hurn, B.A. et al. (1980) in Meth. in Enzymology, Ed. Van Vurakis, H. and Langone, J., pp. 104-142, can be used.

The preparation of monoclonal antibodies is known and if monoclonal antibodies are to be used in this invention, they are prepared using the method originally authored by Milstein and Kohler and published in Nature (1975), 256, pps. 495-497. The basic process involves injecting an animal, usually a mouse, with an immunogenic substance. After suitable time for antibody production to the immunogen, the mouse is sacrificed. Cells are removed from the spleen and fused with myeloma cells. Hybridoma cells resulting from this fusion are able to reproduce in vitro , and each expresses genetic information for one specific antibody. The antibodies produced from one hybridoma fusion thus will only recognize a single antigenic determinant of e the immunogen.

Cells cultured from individual hybridoma cells are screened for production of antibodies to the target antigenic determinant. Those hybridomas positive for the target antigen are further screened to identify those having the highest affinity. The monoclonal antibodies used in the present invention will have an affinity of at least $10^8$ liters/mole. Monoclonal antibodies displaying all of these characteristics are then screened using actual assay conditions to determine if the assay condition alters the antibody binding characteristics or affinity, and to screen out those with cross reactivity to possible contaminating antigens.

In Step 2 of the assay method of the invention, one antibody or set of antibodies is coupled to the solid support matrix and is used to capture Listeria bacterial cells from a sample. The antibody/s used for this step of the assay method are directed against cell surface antigens of the Listeria bacterial cells and, depending upon the degree of specificity desired, may be polyclonal or monoclonal antibodies. Polyclonal antibodies are raised against intact attenuated Listeria bacterial cells. Polyclonal sera with titers approaching $Log_3 = 13$, against Listeria bacteria of the immunogenic strain, have been produced with this method.

Monoclonal antibodies can also be produced, utilizing the methods outlined above. These offer the opportunity to specifically capture desired strains of Listeria bacteria. Antigens used for immunizing mice can be attenuated whole Listeria bacterial cells of the desired strains or specific cell surface antigens.

Monoclonal antibodies directed against Listeria cell surface antigens are made by first culturing cells. Attenuated extracts of these cells are then used to immunize mice and the standard procedure for hybridoma production and monoclonal antibody cloning and screening is followed as described above.

Monoclonal antibodies directed against Listeria cell wall teichoic acids are also produced by methods familiar to those in the field. Immunization is carried out using Listeria cell wall preparations made by the method of Schleifer, K.H. and Handler, O.; (1967) Arch. Mikrobiol. 57, 335-363. These cell wall preparations contain (TA) covalently linked to the cell wall peptidoglycan matrix. For immunization the peptidoglycan will act as the immunogenic carrier. Greater antibody specificity may be achieved by using purified (TA) as the immunizing agent. For this, the cell walls may be digested chemically (for example with 10 mM glycine hydrochloride buffer pH 2.5). The (TA) is then purified and used as the immunogen. To increase the immune response these purified (TA) may be coupled to an immunogenic carrier protein. For example, the reducing sugar end-group of the digested (TA), as above, may be coupled to a carrier protein by reductive amination using the method of Roy et al. (1984) Canadian J. of Biochem., 62, 270-275, as applied to Listeria (TA) by Kamisango et al. (1985), J. Clin. Microbiol., 21, 135-137. In this procedure (TA) are reacted with a carrier protein such as BSA in the presence of sodium cyanoborohydride. The result is a covalent teichoic acid-protein complex which is used for immunization.

Monoclonal antibodies directed against cell wall peptidoglycan (PEP) are made by preparing cell walls as above. Treatment of the cell walls with 10 mM glycine hydrochloride buffer, pH 2.5, followed by centrifugation results in the separation of (TA) into the supernatant and insoluble (PEP) in the pellet. This pellet is resuspended and used as the immunogen. Also possible is further purification of the (PEP) by HPLC or electrophoresis, and the use of purified cleavage fragments from the (PEP) matrix. Using fragments of (PEP) as immunogens was demonstrated by Wergeland, H.I., et al. (1987) J. of Immunol. Meth., 104, 57-63.

Monoclonal antibodies to the cell wall peptidoglycan can also be prepared according to the procedure described in U.S. Patent No. 4,596,769 to Schockman et al.

The thus prepared antibodies are utilized according to the invention in an assay to selectively capture Listeria cells from a sample to be tested, and then potentially to selectively identify the presence of one or more strains of Listeria. The assay can be performed according to various types of assay procedures. In particular, the preferred method of capturing the Listeria bacterial cells is by the use of antibodies coupled to microspheres or magnetic particles. However, any other method able to utilize the immobilized antibodies to capture and concentrate the bacterial cells is feasible. This would include the use of suspended microspheres of latex, polyacrylamide, agarose or other dextrans, etc., in liquified samples to capture the Listeria bacterial cells, followed by centrifugation to concentrate the target Listeria cells. Also included is the collection of target Listeria cells on sticks or paddles coated with antibodies and stirred through liquid or liquified samples. In addition, methods utilizing filtration of the sample through membranes of nitrocellulose, cellulose acetate, nylon or any other substance having antibodies attached is included. Also included is collection of target Listeria bacterial cells by affinity chromatography.

The preferred immobilizing matricies for the capturing antibodies are magnetic beads or polyacrylamide beads. However, any other matrix for immobilizing antibodies is feasible. These include beads of agarose or other polysaccharides, cross-linked dextrans, glass beads, latex beads, glass fiber filters, cellulose nitrate filters and nylon filters. Methods to bind antibodies to polyacrylamide beads and other matrices are well known to those in the profession. Carbodiimides may be used to couple antibodies to polyacrylamide beads as described by Bauminger, J., and Wilchek, M. 1980, Methods of Enzymology, 70, 151-159. Another example is a method for generally binding biopolymers to polysaccharides, U.S. Patent No. 3,645,852.

In one preferred form of the invention the antibodies are immobilized on magnetic beads. This can be accomplished by procedures which are per se known, such as those described in U.S. Patent No. 3,970,518; No. 4,018,886, No. 4,855,045 and No. 4,230,685. In a particular preferred embodiment, attachment of antibodies to magnetic particles is accomplished through a Protein A intermediate. That is, Protein A is first attached to the magnetic particles and the antibodies of choice are then bound to the Protein A. The use of the Protein A intermediate greatly increases the effectiveness of capture by the attached antibodies. (Forsgren et al. (1977) J. Immunol. 99:19) Protein A attaches to the Fc portion of IgG subclass antibodies, thus extending and presenting the Fab portion of these antibodies. The resulting correct orientation of the antibodies and extension away from the particles leads to a very effective interaction between the bound antibodies and their target.

The method of attachment of Protein A to magnetic particles may proceed by any of several processes available through the scientific literature. In one such procedure, magnetic iron oxide particles of approximately 1 micrometer diameter are chemically derivatized by reaction, first with 3-aminopropyltriethoxysilane, then with glutaraldehyde. The derivatized magnetic particles are then mixed with Protein A resulting in a magnetic particle to which Protein A is covalently attached. The antibodies are now added to the Protein A magnetic particles and after a short incubation the protein A-antibody complexes form. (Weetall, H.H.

(1976) Meth. in Enzymol. 44:134-148) These derivatized particles with Protein A-antibodies attached are now ready for use in bacterial cell capture.

Step 3: Release of Listeria Cells

The captured and antibody immobilized Listeria bacterial cells are released from the immobilisation matrix unless the solid support consists of microspheres. In this step the Listeria bacterial cells concentrated from the food or environmental sample must be processed in such a way as to be compatible with the macrophage-like cells in culture (Step 4 below). In the preferred method of the invention the solid support matrix used for capturing the Listeria bacteria is microspheres of magnetic particles. If this support matrix or microspheres of another substance, such as latex, is used the washed antibody bound Listeria bacterial cells can be added directly to a microculture of macrophage-like cells. If the solid support matrix is made of macro particles or filter membrane material it is necessary to remove the cells from this matrix prior to addition to the macrophage-like cell microculture. Methods for removing the Listeria bacterial cells from these solid supports include the use of shifting pH, proteolytic enzymes, salt, detergents, etc. as described in Antibodies, A Laboratory Manual, (1988) Harlow, E. and Lane, A. Eds., Cold Spring Harbor, p. 549.

Step 4: Phagocytization of Listeria by Macrophage-Like Cells

As noted above, one characteristic feature of the method of the invention is the use of macrophage-like cells to selectively culture potentially pathogenic Listeria bacterial cells. Bacteria of the genus Listeria are intracellular parasites of a class of such parasites which grow, feed and replicate within the cytoplasm of their host cells. Once phagocytized by the host cells, in order to escape the vacuolar membrane and subsequently feed, grow and replicate, these bacteria must produce an enzyme (hemolysin) to digest the vacuolar membrane. Once inside the cellular cytoplasm, in order to be pathogenic, these bacteria must also be capable of being transmitted from cell to cell. Pathogenic strains of Listeria accomplish this by mobilizing the host cells polymerized actin filaments in such a way as to propel the Listeria bacteria (encased within the host cells cytoplasmic membrane) into adjacent host cells. The resultant transferred Listeria bacterial cells are now encased by both the vacuolar membrane of the present host cell as well as the cytoplasmic membrane of the previous host cell. The Listeria bacteria then produce and release hemolysin, escape from the membranes into the new host cytoplasm and begin to grow and replicate. Thus the cycle repeats and the Listeria cell number increases with a doubling time of approximately 50 minutes. All of this growth occurs without the Listeria ever leaving the confines of the host macrophage-like cells. (Tilney, L.G. and Portnoy, D.A. (1989) J. of Cell Biol., 109, 1597-1608) Potentially any Listeria target cell capable of being cultured in vitro can be used for the method of the invention. An example of macrophage-like cells used is the mouse J774 cell line. (Ralph, P.J. et al. (1975) J. Immunol., 114, 898-905).

In summary the use of these macrophage-like cells for the detection of potentially pathogenic Listeria bacterial strains offer the following advantages over present art methods: 1) The specificity of growth, replication and cell transmission of intracellular parasites, such as potentially pathogenic Listeria strains, capable of entering the host cellular cytoplasm. This characteristic coupled to a bacterial cell capture method selective for Listeria species results in a high degree of specificity for potentially pathogenic Listeria bacterial strains. 2) An enriched growth media. With very few competitor microorganisms capable of entering the cellular cytoplasm, growing, replicating and being transmitted to other cells the Listeria bacteria originally captured on the solid support matrix can grow rapidly, with growth characteristics similar to growth by a pure Listeria culture in enriched growth media. 3) These "culture" steps within the macrophage-like cells do not require other or additional culture steps nor dilution of sample steps as discussed earlier. 4) Culture of the bacterial cells within the macrophage-like cells are in effect micro cultures and therefore all of the Listeria bacterial cells are assayed for as opposed to a small fraction of the final culture media. 5) Detection of Listeria cells can be carried out by a variety of methods (discussed below) including but not limited by a potentially extremely sensitive microscopic visualization of foci (microcolonies) of potentially pathogenic Listeria bacterial cells radiating from a single infected macrophage-like cell.

Due to these improvements the method of the invention allows for detection of potentially pathogenic Listeria strains from samples in a much sorter time period than existing prior art methods; possibly eight hours or less.

Various macrophage-like cells can be utilized in the assay of the invention including Murine tumor lines, J 774 (ATCC-TIB67) (Portnoy, D.A. et al. (1988), J. Exp. Med., 167, 1459-1471); P388D1 (ATCC-CCL46) (Koren, H.S. et al. (1925) J. Immunol. 114, 894-897) and RAW 264.7 (ATCC-TIB71) (Raschke, W.C. et al. (1978) Cell, 15, 261-267). By the term "macrophage-like cells" are meant those cells in the art to have the characteristics of macrophages (See, for example, Van Furth et al., Cell. Immun; 90, 339-357 (1985). For purposes of this invention, such macrophage-like cells should have the following characteristics:

1) be capable of ingesting Listeria;

2) permit internal or intracellular growth of Listeria; and

3) permit transfer of Listeria from one cell to another, particularly transfer from cell to cell without leaving the host's cytoplasm, remaining intracellular. Depending upon the method of detection utilized, these microcultures of macrophage-like cells can be in uni or multiwell plastic plates, such as microtiter plates used for standard ELISA assays or on glass microscopic cover slips immersed in culture dishes containing an appropriate cell growth medium. The macrophage-like cells attach to the surface of the glass or plastic. In the preferred method of the invention Listeria cells bound to antibody coated magnetic microspheres can be more effectively brought into contact with the glass or plastic attached macrophage-like cells between the magnetic particle bound Listeria bacteria and a magnetic field during a short initial incubation period. Listeria bacterial cells coming in contact with the macrophage-like cells are phagocytized.

Thus, in the assay of the invention, after the Listeria cells (released from the matrix bound antibodies or still bound to the microsphere bound antibodies) are added to a culture of macrophage-like cells, the culture is incubated to allow the macrophage-like cells to phagocytize the Listeria cells. Incubation can be performed according to per se known techniques, such as in DMI medium at 30-40° C (preferably 37° C), at high humidity and with 5% $CO_2$ concentration.

Step 5: Selection of macrophage-like cells containing Listeria

After sufficient incubation, typically for a length of 1/2 hour to 2 hours, the growth medium is removed and replaced with a medium which contains antibiotics to remove bacteria not internalized by macrophage-like cells. Various per se known antibiotics can be used, such as gentamicin, nalidixic acid, penicillin and streptomycin.

Step 6: Once inside the macrophage-like vacuoles, the Listeria bacteria produce an enzyme (hemolysin) which digests the vacuolar membrane and releases the Listeria into the cell cytoplasm. Hemolysin is only produced by certain species of Listeria, including L. monocytogenes. In order to be pathogenic, the Listeria bacteria must produce hemolysin. In addition, they must be invasive to successive target cells. Thus, only potentially pathogenic Listeria will escape the cellular vacuole, grow, reproduce and transfer to adjacent cells.

Pathogenic strains of Listeria accomplish this cell transfer by mobilizing the host cells polymerized actin filaments in such a way as to propel the cellular membrane encased Listeria bacteria into adjacent host cells which in turn phagocytize the end of the Listeria encased cellular projection. The Listeria bacteria grow and reproduce in the newly invaded cell. This cycle continues with the result being an increase in the Listeria bacteria cell number. The doubling time is approximately 50 minutes. Therefore within 8 hours of the original infection the Listeria bacterial cell number has increased by a factor of 1000.

Step 7: The culture medium is now removed leaving the macrophage-like cells attached to the glass or plastic surface and the Listeria bacteria within the cellular cytoplasm.

## Step 8: Disruption of Macrophage-like Cells

The culture media once removed leaves the macrophage-like cells attached to the glass or plastic container surface with the Listeria bacteria within the cellular cytoplasm. Detecting the Listeria then requires that the Listeria cells be released from the macrophage-like cells. To accomplish this, water or methanol is added to the cell-containing dish in an amount simply sufficient to immerse the cells. This disrupts the macrophage-like cells, releasing the captured Listeria which can then be detected, or when methanol used fixes the macrophage-like cells and Listeria to the surface for subsequent detection.

## Detection of Listeria Cells

After the Listeria bacteria are released from the macrophage-like cells, the presence of Listeria can be detected by the use of various means, depending upon the degree of specificity desired, and the Listeria component used as the detection target. It is possible to use direct microscopic examination of fixed and stained macrophage-like cells for the presence of microcolonies of Listeria. Preferably, detection is by use of antibodies to Listeria cells or cellular components, such as organized filaments of polymerized actin.

If antibodies are used for detection of the captured Listeria bacterial cells either polyclonal or monoclonal antibodies can be used. These antibodies are labeled either directly or indirectly with detector molecules. Again, the choice of polyclonal or monoclonal antibodies is determined by the degree of specificity to Listeria strains desired and the binding characteristics of the antibodies to the target Listeria bacterial cells. Also, monoclonal antibodies raised against cellular filamentous actin may be used to detect captured Listeria bacteria via the linkage of the organized filamentous actin net associated with the Listeria bacteria growing, replicating and being transmitted between host macrophage-like cells. Labeled phalloidin (an actin binding toxin) or labeled myosin may also be used in place of anti-actin antibodies.

In addition to utilizing directly or indirectly labeled antibodies to detect captured, and macrophage-like cell cultured, bacteria or the filamentous actin net associated with these Listeria bacteria it is also possible to detect the Listeria bacterial cells by direct visualization of the stained microscopic slides. In this application of the assay method after infecting the macrophage-like cells on a glass cover slip, with the Listeria captured from the sample, and allowing the Listeria bacteria to grow, replicate and be transmitted to nearby cells, the glass cover slip is fixed and stained to highlight Listeria bacterial cells. Foci, or microcolonies of Listeria bacterial cells will be evident upon microscopic examination of the glass cover slip if originally present in the sample, captured on the solid support and internalized by the macrophage-like cells.

Actual detection of Listeria bacteria can follow a number of methods. A flow chart outlining these methods is shown in (figure 4).

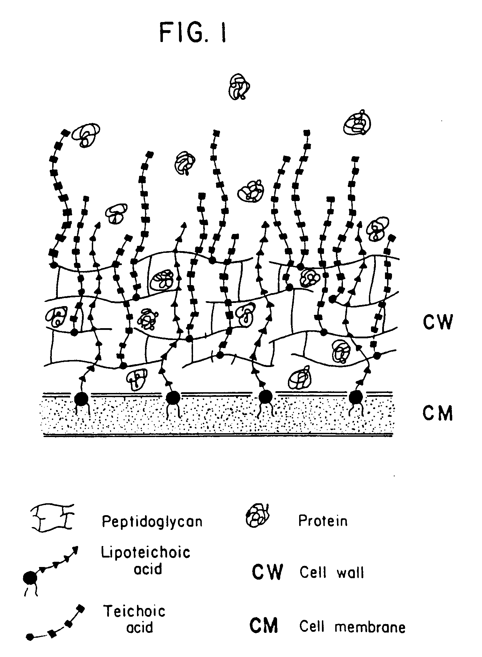

One preferred method according to the invention for detecting the presence of Listeria takes advantage of the particular structure characteristics of Listeria cell walls, particularly the use of the peptidoglycan (PEP) and teichoic acid (TA) moieties, and specifically the peptidoglycan-teichoic acid (PEP-TA) complex as a capture-target antigen group to selectively detect the presence of Listeria strains, taking advantage of the fact that Listeria strains contain a number of differing teichoic acids. The cell wall of Listeria strains is composed of diaminopimelic type peptidoglycan, ribitol containing teichoic acid, lipoteichoic acid and protein. The general organization of these cell wall components was described by Fiedler, F. (1988) in Infection 16, Suppl. 2, pp. 392-297 and is shown in Figure 1.

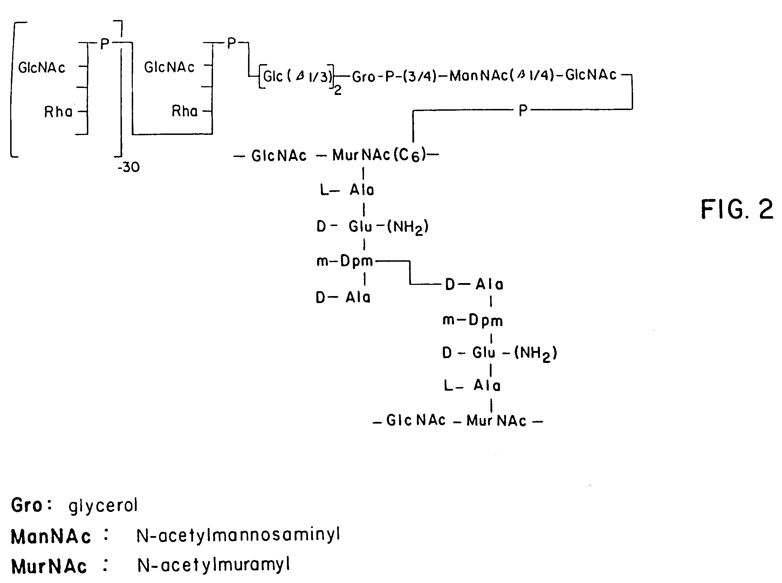

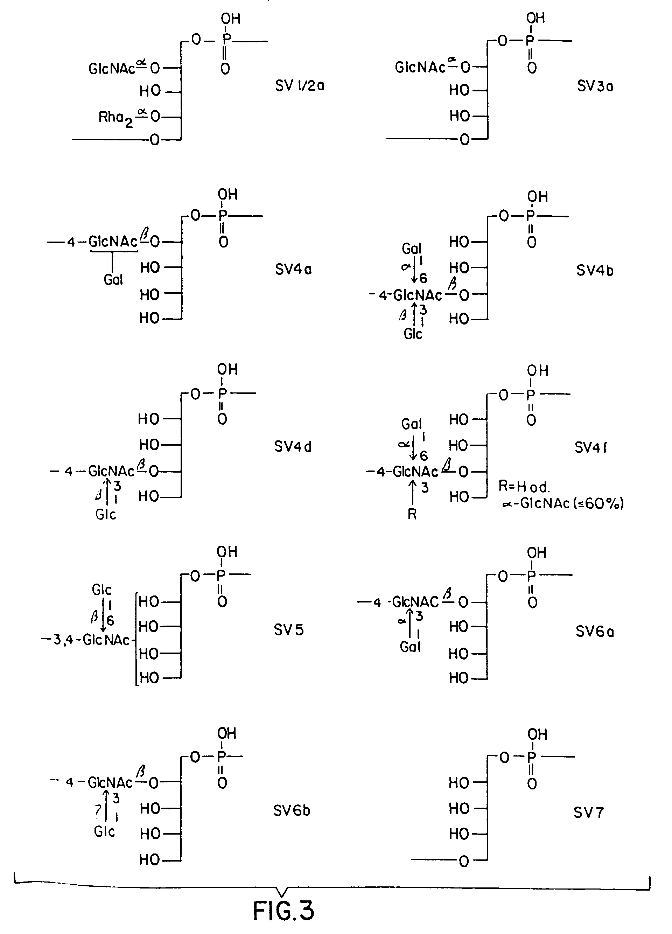

As can be seen in Figure 2, the cell wall includes a peptidoglycan matrix to which are bound a large number of teichoic acid molecules. An example of the chemical structure of a portion of the peptidoglycan matrix covalently attached to an individual teichoic acid molecule in one Listeria strain is shown in Figure 3. The teichoic acid molecule includes a sugar moiety comprised of ribitol or a substituted ribitol. In a single teichoic acid molecule, an individual ribitol or substituted ribitol is repeated approximately 30 times. In addition, there are differences in the sugar substitution to the ribitols in different strains of Listeria. These differences in the ribitol substitution among strains of Listeria are shown in Figure 4.

The present invention takes advantage of these structural characteristics of the Listeria bacterial cell wall to provide an assay method which is capable of detecting the presence of Listeria strains, and, if desired, is able to distinguish between strains of Listeria. Since only some Listeria strains are known to be pathogenic it is possible to detect only the pathogenic strains, utilizing to advantage the characteristic teichoic acid structure of those strains.

In particular, with reference to Fig. 4 showing the ribitol substitutions for different Listeria strains, strains SV 1/2a and 4b are the most common pathogenic strains; SV 5 is primarily an animal pathogen, and the remaining strains are rarely pathogenic.

Detection and selection of the bacteria and cell wall components is accomplished by the use of antibodies and can be performed by methods using either polyclonal antibodies or monoclonal antibodies in the individual steps of the assay, depending upon various factors, including the degree of sensitivity desired.

The preferred detection method, therefore, generally comprises the following procedural steps:

1) After disruption of the macrophage-like cells to release Listeria cells, the released Listeria cells are treated (preferably with mutanolysin) to release target antigens, i.e. peptidoglycan-teichoic acid complexes, from the cell walls.

2) The thus treated complexes are combined, such as by passing through an affinity column, with an affinity matrix having bound thereto antibodies to the peptidoglycan (PEP) moiety, whereby the peptidoglycan moiety is captured by the antibodies.

3) A solution containing labeled antibodies to the teichoic acid (TA) moiety of the PEP-TA complex is combined with the column matrix whereby these labeled antibodies bind to the teichoic acid portion of the captured PEP-TA complexes.

4) The affinity matrix is then treated with a releasing agent to release the label from the matrix, or break any part of the linkage to the matrix; and the presence of labeled monoclonal antibodies is detected as a direct indicator of the presence of the bacterial pathogen.

The detection procedure then begins after the macrophage-like cells are disrupted and the Listeria cells are released, and a solution of mutanolysin is added which disrupts the Listeria cell walls producing specific cell wall components. The product of this digestion is individual and/or multiple units of cell wall peptidoglycan covalently linked to cell wall teichoic acid. These peptidoglycan-teichoic acid complexes (PEP-TA) are the bacterial target antigens for the immunoassay. This solution is removed and applied directly to a column which contains immobilized monoclonal or polyclonal antibodies to the peptidoglycan moiety of the PEP-TA complex covalently attached to a solid support, such as agarose beads. A buffer solution is washed through the column to remove all remaining cellular components except the captured PEP-TA complexes.

A solution containing monoclonal antibodies to Listeria teichoic acid (TA) is then added to the column. These antibodies are specific to Listeria strains. As noted above and shown in Figure 4, the structure of (TA) varies among the strains of Listeria and, thus, more than one antibody is required for detection of the different strains of Listeria. If any selected strains are to be identified, the antibodies to any of these strains can be used. These antibodies are labeled directly, or indirectly with detector molecules.

After passing this solution over the column, the column is washed thoroughly to remove all labeled antibody not specifically attached to the TA moiety of the PEP-TA complex.

Final specificity of the test is accomplished at this step of the procedure.

A small volume of a releasing solution is then passed over the column to release the detector molecules attached to the TA antibodies, or labeled TA antibodies. The releasing solution containing the released detector molecules is collected and the amount of label is measured. This measurement can either be a direct indicator of the presence of Listeria strains or can be compared against a set of control columns.

Instead of detecting Listeria cell wall components, it is also possible to detect Listeria by the presence of hemolysin. In this case, antibodies to hemolysin would be utilized and substituted for the peptidoglycan antibodies.

If an affinity column containing monoclonal antibodies to Listeria produced hemolysin is substituted for the peptidoglycan antibody described above, after lysing the macrophage-like cells with water, the lysed cells and solution are applied to the affinity column. Then, after washing, a labeled anti-hemolysin monoclonal detector antibody (with affinity to a different epitope) is applied to the column. Detection of the label then proceeds as described below.

Another modification to the present invention would add anti-Listeria bound magnetic microspheres to the solution containing the water lysed macrophage-like cells to capture the Listeria bacterial cells. These captured cells would then be separated from the solution by means of a magnetic field. For example, this separation can proceed by placing the magnetic field beneath the micro plate used for the earlier steps involving the macrophage-like cells. The separated Listeria bacterial cells attached to the magnetic particles would then be washed. Finally detection of the cells can follow by the methods outlined above or the actin net produced by the Listeria bacterial cells during the infection and cell transfer process can be detected by labeled anti actin monoclonal antibodies, labeled actin binding myosin fragments, or with labeled phalloidin (an actin binding toxin). Detection of the actin net offers a large amplification of signal due to the repeating nature of the actin molecule and the amount of actin attached to the Listeria bacterial cells.

If the macrophage-like cells are treated with methanol these cells and the Listeria bacteria become fixed to the surface of the plastic or glass. At this point any available detection scheme for bacterial cells on microscopic slides may be utilized. For example fluorescent labeled antibodies to Listeria bacterial cell surface can be applied to the glass slip and, after an incubation and wash step, microscopic examination of the slip will detect Listeria as fluorescent foci, representing microcolonies of Listeria bacterial cells. If the Listeria bacterial cells were fixed onto the surface of microtiter plates enzyme labeled antibodies can be added and bound to the Listeria cell surface. Then depending upon the substrate added, either a fluorescent, colorometric or other form of product might be detected in solution.

In the above described procedure, antibodies directed against the Listeria bacterial cell surface serve as the signal or detector antibodies. These are labeled either directly or indirectly with labels used in other known immunoassays. Direct labels (labels attached directly to the anti-Listeria cell surface antibodies) may include fluorogenic, chemiluminescent, bioluminescent, radioactive, metallic, biotin or enzymatic molecules. Methods of combining these labels to antibodies or other macromolecules are well known to those in the art. Examples include the methods of Hijmans, W. et al. (1969), Clin. Exp. Immunol. 4, 457-, for fluorescein isothiocyanate, the method of Goding, J.W. (1976), J. Immunol. Meth. 13, 215-, for tetramethylrhodamine isothiocyanate, and the method of Engrall, E. (1980), Meth. in Enzymol. 70, 419-439 for enzymes.

These detector antibodies may also be labeled indirectly. In this case the actual detection molecule is attached to a secondary antibody or other molecule with binding affinity for the anti-Listeria cell surface antibody. If a secondary antibody is used it is preferably a general antibody to a class of antibody from the animal species used to raise the anti-Listeria cell surface antibodies. Also, in the method of the invention described above, the labeled detector molecule may be a molecule having binding affinity to polymerized actin if this embodiment of the method described above is utilized.

**Claims**

1. A method for the detection of Listeria strains which comprises:
   a) incubating a mixture of Listeria cells and macrophage-like cells to permit said macrophage-like cells to phagocytize said Listeria and to permit said Listeria to reproduce within said macrophage-like cells;
   b) disrupting said macrophage-like cells to release said Listeria; and
   c) detecting the presence of said Listeria.

2. A method for the detection of Listeria strains which comprises:
   a) capturing Listeria cells from a sample potentially containing Listeria;
   b) combining and incubating said captured Listeria cells with macrophage-like cells to permit said macrophage-like cells to phagocytize said Listeria and to permit said Listeria cells to reproduce;
   c) disrupting said macrophage-like cells to release said phagocytized Listeria cells; and
   d) detecting the presence of Listeria.

3. A method for the detection of Listeria strains which comprises:
   a) capturing Listeria cells from a sample potentially containing Listeria strains;
   b) combining and incubating in a vessel said captured Listeria cells with macrophage-like cells to permit said macrophage-like cells to phagocytize said Listeria and to permit said Listeria cells to reproduce;
   c) fixing said macrophage-like cells containing phagocytized Listeria cells to the surface of said vessel; and
   d) detecting the presence of Listeria.

4. A method according to either claim 2 or 3, wherein said Listeria cells are captured from said sample by exposing said sample to a solid support having immobilized thereon antibodies to Listeria cells.

5. A method according to claim 4, wherein said solid support comprises magnetic beads.

6. The method according to any one of claims 1 to 5, wherein said macrophage-like cells are selected from the group consisting of the cell lines J774 (ATCC-TIB67), P388D1 (ATCC-CCL46) and RAW 264.7 (ATCC-TIB71).

7. A method according to claim 1 or 2, wherein said macrophage-like cells are disrupted by treatment with water.

8. The method according to claim 3, wherein said macrophage-like cells are fixed by treatment with methanol.

9. The method according to claim 8, wherein following fixation of said macrophage-like cells, the presence of Listeria cells is determined by microscopic examination.

10. The method according to claim 1 or 2, wherein following disruption of said macrophage-like cells, the presence of Listeria cells is determined by detecting the presence of Listeria cell wall components, hemolysin or filaments of polymerized actin.

11. The method according to claim 3, wherein following fixing of said macrophage-like cells, the presence of Listeria cells is determined by treating said fixed cells with labeled antibodies to Listeria cells, Listeria cell wall components or filaments of polymerized actin.

12. The method according to claim 10, wherein following disruption of said macrophage-like cells, released Listeria cells are recaptured by combining said disrupted macrophage-like cells and released Listeria cells with a solid support having immobilized thereon antibodies to Listeria cells, and subsequently detecting the presence of recaptured Listeria cells by detecting the presence of Listeria cell wall components or filaments of polymerized actin.

13. The method according to claim 12, wherein the detection of said Listeria cell wall components is performed by
    a) treating said recaptured Listeria cells to release peptidoglycan-teichoic acid (PEP-TA) complexes from said Listeria cells;
    b) combining said PEP-TA complexes with antibodies specific for peptidoglycan, to thereby capture said PEP-TA complexes;
    c) combining said captured PEP-TA complexes with labeled antibodies to teichoic acid, to thereby bind said labeled antibodies to said captured PEP-TA complexes;
    d) treating the thus produced PEP-TA-labeled antibody complex to release said label; and

    e) measuring said label to detect or measure the presence of Listeria strains in the sample

14. The method according to claim 13, wherein said treatment of said recaptured cells is performed with lytic enzymes.

15. The method according to claim 10, wherein the detection of said Listeria cell wall components is performed by
    a) treating said released Listeria cells to release peptidoglycan-teichoic acid (PEP-TA) complexes from said Listeria cells;
    b) combining said PEP-TA complexes with antibodies specific for peptidoglycan, to thereby capture said PEP-TA complexes;
    c) combining said captured PEP-TA complexes with labeled antibodies to teichoic acid, to thereby bind said labeled antibodies to said captured PEP-TA complexes;
    d) treating the thus produced PEP-TA-labeled antibody complex to release said label; and
    e) measuring said label to detect or measure the presence of Listeria strains in the sample.

16. The method according to claim 15, wherein said treatment of said released Listeria cells is performed with lytic enzymes.

17. The method of either claim 14 or 16, wherein said lytic enzyme is mutanolysin.

18. The method according to any one of claims 13 to 17, wherein said antibodies to teichoic acid are specific to teichoic acid found in pathogenic Listeria strains.

19. The method according to any one of claims 11 to 18, wherein said labeled antibodies are labeled with fluorogenic, chemiluminescent, bioluminescent, radioactive, metallic, biotin or enzymatic molecules.

20. A method for the detection of Listeria strains which comprises:
    a) combining a sample potentially containing Listeria strains with a solid support having immobilized thereon antibodies to Listeria cells, to thereby capture Listeria cells from said sample;
    b) combining said captured Listeria cells with macrophage-like cells capable of phagocytizing said Listeria;
    c) incubating said combination of Listeria cells and macrophage-like cells for a time sufficient to permit said macrophage-like cells to phagocytize said Listeria;

d) treating said combination with antibiotics to kill bacteria not phagocytized by said macrophage-like cells;

e) incubating said combination in a vessel to permit said Listeria cells to reproduce;

f) disrupting said macrophage-like cells to release said phagocytized Listeria cells or fixing said macrophage-like cells to the surface of said vessel; and

g) detecting the presence of Listeria.

21. The method according to claim 20, wherein said solid support comprises magnetic beads.

22. A method according to claim 20 or 21, wherein said macrophage-like cells are disrupted by treatment with water or fixed by treatment with methanol.

23. The method according to any one of claims 20 to 22, wherein said macrophage-like cells are selected from the group consisting of the cell lines J774 (ATCC-TIB67), P388D1 (ATCC-CCL46) and RAW 264.7 (ATCC-TIB71).

24. The method according to any one of claims 20 to 23, wherein following fixing of said macrophage-like cells, the presence of Listeria cells is determined by microscopic examination or by treating said fixed cells with antibodies to Listeria cells, cell wall components or filaments of polymerized actin.

25. The method according to any one of claims 20 to 23, wherein following disruption of said macrophage-like cells, the presence of Listeria cells is determined by detecting the presence of Listeria cell wall components, hemolysin or filaments of polymerized actin.

# FIG. I

| | Peptidoglycan | | Protein |
| --- | --- | --- | --- |
| | Lipoteichoic acid | CW | Cell wall |
| | Teichoic acid | CM | Cell membrane |

EP 0 463 222 A2

FIG. 2

$$\left[\begin{array}{c} \text{GlcNAc} \\ | \\ \text{Rha} \end{array}\right]\text{-P}\quad \left[\text{GlcNAc}\right]\text{-P}$$

Structure:

```
┌GlcNAc─┐P┐   ┌GlcNAc─┐P─┐
│   │   │ │   │   │    │  │
│  Rha─ │ │   │  Rha─  │  │
└───────┘ │   └────────┘  │
          └──────────┐    │
                   -30
```

[Glc ( β 1/3)]₂—Gro-P-(3/4)—ManNAc( β 1/4)-GlcNAc

— GlcNAc — MurNAc(C₆)—
                |
             L— Ala
                |
          D— Glu—(NH₂)
                |
            m—Dpm ——— D—Ala
                |            |
             D— Ala      m—Dpm
                             |
                        D— Glu —(NH₂)
                             |
                          L— Ala
                             |
              — GlcNAc — MurNAc —

P

**Gro :** glycerol

**ManNAc :**  N-acetylmannosaminyl

**MurNAc :**  N-acetylmuramyl

FIG.3

# FIG. 4

Methods Described in Text to Assay For Viable Potentially
Pathogenic Listeria.

Prepare Sample
↓
Capture Listeria
(attach to solid support)
↓
Separate Capture, Listeria
from Sample Mixture and Wash
↓
Release Listeria From Solid
Support, if Necessary
↓
Apply Listeria to Macrophage-Like Cell
Culture
↓

Stain and
Detect Listeria  ←─ Amplify Viable and Potentially Pathogenic
Microcolonies        Listeria Within Macrophage-Like Cells
by Direct                           ↓
Microscopic         Disrupt Macrophage-Like Cells
Exam                to Release Listeria

With Water                                    With Methanol
                                              (fixes Listeria to
                                               glass or plastic
                                               surface)

              Recapture Listeria
              on solid support

Assay By                    Assay By                    Assay By
1) Affinity chrom-          1) Affinity chrom-          1) Microscopic
atography of digested       atography of digested       exam of floures-
cells wall components       cell wall components         cent antibodies
2) Affinity chrom-          2) Sandwich assay to        bound to Listeria
atography of Listeria       Listeria cell attached      microcolonies
produced hemolysin          actin net                   2) Well assays
                                                         using antibodies
                                                         with labels
                                                         producing
                                                         soluable products

16